# EUROPEAN PATENT SPECIFICATION

(11) **EP 3 134 133 B1**
(45) Date of publication and mention of the grant of the patent: **15.08.2018**
(21) Application number: 15720437.1
(22) Date of filing: 26.03.2015
(51) Int. Cl.: A61L 2/025, A61L 2/04, B08B 3/02, B08B 3/10, B08B 3/12, A61C 19/00

(54) **ULTRASONIC THERMAL DISINFECTOR**
ULTRASCHALL-WÄRMEDESINFEKTOR
DÉSINFECTEUR THERMIQUE ULTRASONORE

(30) Priority: 02.04.2014 IT BO20140185
(43) Date of publication of application: 01.03.2017
(73) Proprietor: CEFLA Società Cooperativa, 40026 Imola (BO) (IT)
(72) Inventor: BELLOSI, Angelo, I-40026 Imola (Bologna) (IT); TOSI, Daniele, I-27018 Vidigulfo (Pavia) (IT); ALBERTAZZI, Chiara, I-48020 Sant'agata sul Santerno (Ravenna) (IT)
(74) Representative: Lunati & Mazzoni S.r.L.
(86) International application number: PCT/IB2015/052226
(87) International publication number: WO 2015/150986

(56) References cited:
- CN-A- 1 279 113
- CN-Y- 201 423 361
- FR-A1- 2 715 876
- US-A1- 2002 159 917
- US-A1- 2011 132 404
- US-B2- 7 803 315

## Description

The present invention relates to a thermal disinfector provided with ultrasonic cleansing for the disinfection of dental instruments, or more generally, of medical/surgical instruments.

Generally, the gold standard in the treatment of medical/dental instruments is the sterilization through water steam autoclave; the efficacy of the sterilizing cycle is however linked to the state of cleanliness of the instruments which are inserted into the autoclave. Therefore, pre-treating the instruments before wrapping and inserting them into the autoclave is a common practice.

In the art two different apparatuses to pre-treat instruments to be sterilized have been known for a long time:
a) Ultrasonic baths for immersing instruments
   These are basins in which the instruments are immersed, where the instruments undergo the action of ultrasounds, which enable the removal of residual solids, especially plaster, dental cements, composite materials, present on the instruments themselves. Specific detergents/disinfectants may be added to the bath water.
b) Thermal disinfectors
   These are apparatuses which have a configuration very similar to traditional domestic dishwashers, which cleanse with water performing a strong mechanical action, at a high temperature (around 90°C). In this case, too, specific detergents may be used.

Ultrasonic baths effectively clean instruments only, while thermal disinfectors ensure also a high degree of disinfection.

Both these apparatuses make it possible to decrease the risk to human operators linked to handling cutting/sharp instruments, and to better standardize the kind of treatment instruments undergo.

The disadvantage linked to thermal disinfector use is the duration of the disinfecting cycle, which, like in domestic dishwashers, is rather long-lasting, around two hours. Moreover, without the ultrasound cleansing, the removal of residual cement from instruments is more difficult, and therefore the operator has to remove it manually.

An apparatus that appeared more recently on the market is described in Soltec's patents IT1404342 and IT1404343. This apparatus is provided with a basin where instruments are immersed and undergo ultrasonic cleansing, with an optional thermal disinfection while the instruments are immersed. This kind of apparatus requires the heating of a large quantity of water, and therefore a long-lasting cycle duration. The basin is provided with a heating coil for heating the water in which the coil is immersed.

Another similar apparatus is described in patent application CN 201 423 361 Y, which describes a basin extending in a mainly vertical direction with heating means arranged on the bottom of and substantially inside said basin.

The present invention aims to provide an apparatus and a method to cleanse and disinfect instruments, improving the duration and the efficiency of the process, and protecting human operators.

This purpose is achieved by an apparatus and a method having the features of the independent claims. Advantageous embodiments and refinements are specified in the claims dependent thereon.

The apparatus of the present invention is as defined in claim 1. With respect to the prior art, the advantage of the present apparatus as compared to ultrasonic baths and thermal disinfectors is the better efficiency of the process. With respect to the apparatus described in Soltec's patents and in patent application CN 201 423 361 Y there is a lower use of water and a more rapid cycle. This is obtained thanks to the special heating system, which does not require the use of heating coils within the basin, and to the fact that the thermal disinfection step is not performed with immersed instruments, but is performed through water jets which recirculate the water present in the basin.

Further advantages and properties of the present invention are disclosed in the following description, in which exemplary embodiments of the present invention are explained in detail based on the drawings:
- **Figure 1**: Perspective view of the thermal disinfector;
- **Figure 2**: Schematic diagram of the thermal disinfector;
- **Figure 3**: Schematic diagram of a further embodiment of the thermal disinfector.

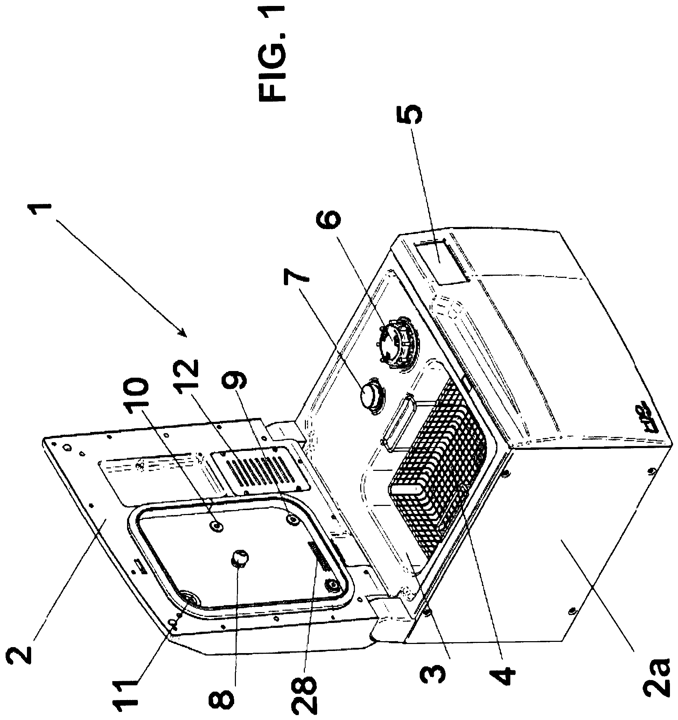

Figure 1 shows a perspective view of the thermal disinfector of the present invention. The thermal disinfector 1 comprises a cover 2, a basin 3, a display 5.

Within the basin 3 there is a basket 4 where the instruments to be washed and disinfected are placed. On the surface beneath the cover 2 there is an inlet 6 to the reservoir of salt for the regeneration of the resins of a water softener, and an inlet 7 to the detergent reservoir.

Structurally the thermal disinfector 1 preferably comprises a container 2a, which preferably houses said basin 3, in a raised position with respect to the bottom of said container 2a, and the other elements. The container 2a is preferably entirely closed by the cover 2.

On the underside of the cover 2 there is at least a sprinkler 8 for inletting water jets into the basin, and also a nozzle 9 for inletting water coming from the water mains. Also on the underside of the cover 2 there is a port 11 for introducing air for the drying step, which flows out through the exit port 28. Under the grid 12 there is a HEPA filter to filter incoming air.

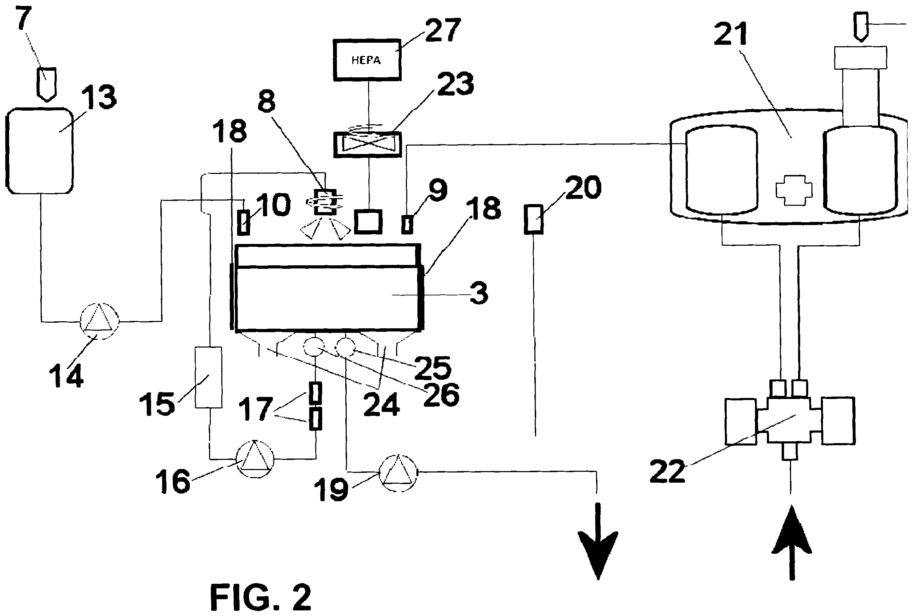

Figure 2 shows a schematic diagram of the components of the thermal disinfector 1. The basin 3 is provided with a plurality of ultrasound generators 24. Moreover, the basin 3 is provided with external heating bands 18, consisting of electric resistors, for heating the walls of the basin 3.

The basin 3 extends preferably mainly in a horizontal plane, in particular the dimensions in the horizontal plane are more than three times greater than the dimensions in the vertical direction. The ultrasound generators 24 are also preferably arranged in correspondence with the largest surface, that is to say in correspondence with the lower surface that extends in the horizontal plane.

The basin 3 can be filled to two different water levels:
- A high level for immersing instruments during the ultrasound step;
- A low level for the initial prewash step, rinsing step and thermal disinfection step.

Water can enter the basin 3 through two different inlets:
- The nozzle 9, introducing water coming from the water mains (indicated with the upward bold arrow) into the basin 3 through a valve 22; said water is softened thanks to the passage through a softener group 21, and enters the basin at the room temperature of the mains water;
- The sprinkler(s) 8, introducing recirculating water only into the basin 3, passing through a recirculating pump 16 and a water heater 15, which can be on or off. When the water heater 15 is off, it does not heat the water.

Incoming water, in any quantity, is always initially introduced through the nozzle 9. The basin 3 is provided with two outlets for water: an outlet 26 which is the outlet for recirculating water, and an outlet 25, which connects the basin to the drain and eventually to the sewer; said final outlet is indicated with the downward bold arrow. On the drainage line from the outlet 25 a connection branches off, at whose end is positioned a level sensor 20 which detects the water level in the basin 3. Also on the drainage line there is a pump 19 for extracting water from the basin 3.

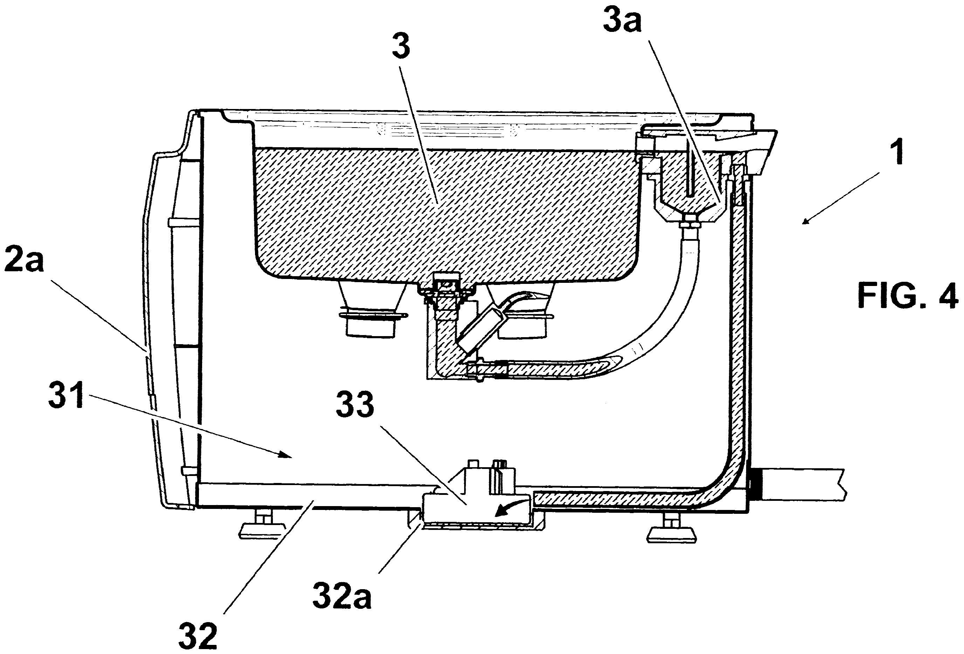

The thermal disinfector 1 also, preferably, comprises anti-flooding means 31 (Fig. 4), preferably comprising a containment tray 32 arranged on the bottom of the container 2a and suitable to contain the water that flows out through broken pipes or from the basin 3 through an overflow hole 3a. The anti-flooding means 31 preferably comprise a float valve 33 arranged in said containment tray 32 and more preferably in a lowered portion 32a of the tray 32. The float valve 33 is preferably mechanically linked to a water shut-off valve, preferably a solenoid valve. Said solenoid valve is appropriately suitable to move and disconnect the water circuit, and the flow of water into the thermal disinfector 1 and the container 2a when the float valve 33 rises due to flooding.

Downstream of the recirculating water outlet 26 there is at least a temperature sensor 17 for detecting the temperature of the recirculating water.

In the thermal disinfector 1 there is a reservoir 13 for a detergent, which through a dosing pump 14 is directly introduced into the basin 3 through a specific inlet 10.

Above the basin 3 there is a fan 23 supplied with air passing through a HEPA filter 27 intended to purify the air entering the basin; said air is used to dry the instruments, and is filtered so that it does not contaminate them in the final step of the cycle successive to the disinfection step. The incoming air also preferably passes through heating means, preferably comprising a labyrinth with heating fins, suitable to heat the air in order to dry the instruments quickly. The air inlet duct preferably also comprises a temperature sensor and a one-way valve that allows the air to enter the basin 3 but not to flow back out of it.

The position of the heater 15 outside and at a distance from the basin 3, and so not directly in contact with the basin 3, avoids the need for heating coils inside the basin, as is the case in the apparatus described in the aforesaid patents. This is advantageous in that it leaves the basin totally free, so that cleansing is easier and the safety of the thermal disinfector 1 is enhanced. This is an advantage also in terms of structural simplicity and reliability, in that the basin 3 does not exhibit holes for the passage of the ends of the resistors, which would require a watertight sealing, difficult to ensure given the presence of the vibrations caused by the sonotrodes 24.

Moreover, the recirculating water is heated as it passes through the water heater 15, and falls in the form of jets through sprinklers 8 on the instruments contained in the basket 4. This allows the use of smaller quantities of water, which are therefore heated more rapidly, significantly reducing the duration of the washing and thermal disinfection cycle.

The sprinklers 8 in one embodiment are present in the form of rotors, like those of a traditional domestic dishwasher; in the preferred embodiment, they are in the form of rotating nozzles. This is different from the fixed nozzles described in the aforesaid patent documents, in that their presence adds a mechanical action that significantly improves the washing and the removal of particles from instruments. Moreover, the sprinklers 8 sprinkle hot water in the basin 3 generating a whirl, thus improving the distribution of heat and detergent. Rotating nozzles generate a better water distribution as compared to rotors, using less water.

In practice, a human operator gathers the dirty instruments, suitably positions them in the basket 4, positions the filled basket 4 in the basin 3 and then closes the cover 2. Then she/he starts the automatic cycle using the display 5. The successive steps of the thermal disinfection cycle are automatically controlled by an electronic control unit (not shown).

The method applied by the ultrasonic thermal disinfector of the present invention comprises the following steps:
a) Introduction of water coming from the mains through the nozzle 9 via the softener group 21;
b) Cold cleansing through the sprinklers 8;
c) Release of dirty water through the outlet 25 towards the sewer;
d) Introduction of water coming from the mains through the nozzle 9 via the softener group 21; water is introduced up to its higher level;
e) Introduction of detergent, coming from the reservoir 13;
f) Heating of the liquid (water and detergent) present in the basin 3; said heating occurs through the combined action of the heating bands 18 and of the outside water heater 15; at the same time ultrasonic washing is started; ultrasounds are generated by sonotrodes 24; thanks to recirculation, the water is progressively brought to about 45°C; this step of ultrasonic washing requires at least 10 minutes;
g) Release of dirty water through the outlet 25 towards the sewer;
h) Introduction of water coming from the mains through the nozzle 9 via the softener group 21; water is introduced to its lower level;
i) Rinsing with clean water through the sprinklers 8 with recirculation of the water;
j) Release of dirty water through the outlet 25 towards the sewer;
k) Introduction of water coming from the mains through the nozzle 9 via the softener group 21; water is introduced up to its lower level;
l) Heating of the water through the combined action of the heating bands and the outside water heater 15;
m)The water is kept at the pre-set temperature for the contact time necessary to obtain thermal disinfection; in the preferred embodiment, at 90°C for at least 5 minutes;
n) Release of dirty water through the outlet 25 towards the sewer;
o) Drying through air entering the basin 3 via the air port 11 passing through the HEPA filter 27; the air is released towards the environment through the air port 28.

In particular, concerning the ultrasonic cleansing step f), water heating is performed using the heating bands 18 and using water recirculation through the outside water heater 15 only for the minimum needed to mix the water and eliminate thermal stratification.

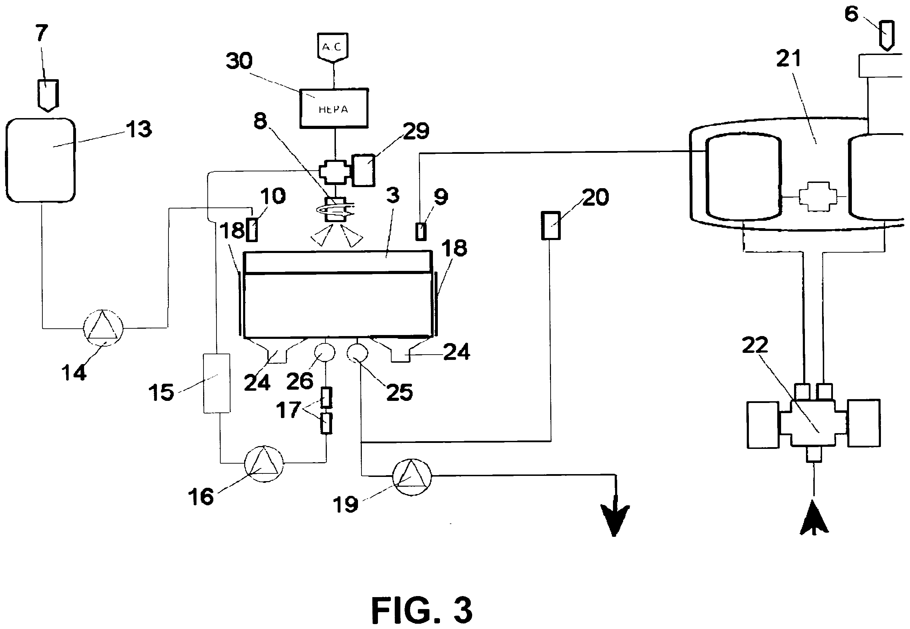

Concerning the drying step o), in a preferred embodiment shown in Figure 3, the air is taken from outside the thermal disinfector 1, passes through a special HEPA filter 30 for compressed air, and is introduced into the basin 3 through the rotors or rotating nozzles 8. This makes it possible to improve air distribution, and more generally the drying step. Obviously the presence of a valve 29 is necessary, to allow the filtered air pipe to be connected to the last portion of the water pipe upstream of the sprinklers 8.

The total time of a cycle performed as described above is around 40 minutes. This is a very short time, if compared to the duration of a cycle of a traditional thermal disinfector, which requires a variable time of between 120-75 minutes. The above-described cycle makes it possible to obtain instruments free of residual solids, thanks to the mechanical action performed by the rotating nozzles and ultrasonic cleansing; moreover the instruments have a contamination level much lower than that obtainable with a simple ultrasonic bath without thermal disinfection.

Finally, the reduced quantity of water used thanks to recirculation allows heating to be performed very rapidly, and this significantly shortens the duration of the thermal disinfection cycle.
- 1.: thermal disinfector
- 2.: cover - 2a container
- 3.: basin - 3a overflow hole
- 4.: basket
- 5.: display
- 6.: salt reservoir inlet
- 7.: detergent reservoir inlet
- 8.: sprinklers
- 9.: water inlet
- 10.: detergent inlet port
- 11.: air inlet port
- 12.: HEPA filter grid
- 13.: detergent reservoir
- 14.: detergent dosing pump
- 15.: heater
- 16.: water recirculating pump
- 17.: temperature sensors
- 18.: heating bands
- 19.: releasing pump
- 20.: water pressure sensor
- 21.: softener group
- 22.: mains water inlet valve
- 23.: drying fan
- 24.: sonotrodes
- 25.: water outlet
- 26.: recirculating water outlet
- 27.: HEPA filter
- 28.: air outlet port
- 29.: exchange valve
- 30.: HEPA filter for compressed air
- 31. 31: anti-flooding means
- 32.: tray - 32a lowered portion
- 33.: float valve

## Claims

1. Thermal disinfector (1) comprising:
- a cover (2),
- a basin (3),
- at least an inlet (8, 9) for the water,
- an outlet (25) to drain the water to the sewer,
- an outlet (26) for water to be recirculated and reintroduced into the basin (3),
- at least one ultrasound generator (24),
- an electronic control unit,
**characterized in that** it comprises
- a water heater (15) positioned outside and at a distance from the basin (3) and so not directly in contact with the basin (3),
- and at least one heating band (18) adhering to the basin (3).

2. Thermal disinfector according to claim 1, wherein the water is introduced into the basin (3) following one of two distinct paths:
- water coming from the mains passes through a softener group (21) and enters the basin (3) through the nozzle (9);
- water contained in the basin (3) flows out through the recirculation outlet (26), passes through the outside water heater (15), enters the basin (3) through at least one sprinkler (8); in said path the external water heater (15) may be on, heating the water, or off, not heating it.

3. Thermal disinfector (1) according to claim 1, wherein at least one of said water inlets (8) transmits a mechanical action to the water, preferably said water inlet (8) is in the form of a rotating nozzle or rotor.

4. Thermal disinfector according to claims 1- 3 further comprising a HEPA filter (27) to filter air coming from the environment, a port (11) to introduce air into the basin (3), and a port (28) for releasing air toward the environment.

5. Thermal disinfector according to the preceding claim, comprising means for heating said air introduced from the environment.

6. Thermal disinfector according to claim 1, further comprising an exchange valve (29) which allows the air for drying the instruments to be conveyed through the rotating nozzles or rotors (8).

7. Thermal disinfector according to one or more of the preceding claims, wherein said basin (3) extends mainly in the horizontal plane.

8. Thermal disinfector according to the preceding claim, wherein said ultrasound generator (24) is preferably arranged in correspondence with the largest surface of said basin (3), that is to say in correspondence with the lower surface which extends in said horizontal plane.

9. Thermal disinfector according to one or more of the preceding claims, comprising a container (2a) housing at least said basin (3) in a raised position, further comprising anti-flooding means (31), comprising a containment tray (32) arranged on the bottom of said container (2a) and comprising a float valve (33) connected to a water shut-off valve, suitable for preventing the water from entering the thermal disinfector (1) when the float valve (33) rises due to flooding.

10. Method for ultrasonic cleansing and thermal disinfection of instruments placed in the basket (4), contained inside the basin (3) of the thermal disinfector (1) comprising the following steps:
a) Pre-wash with cold water;
b) Cleansing of immersed or non-immersed instruments with or without ultrasounds, at a temperature of 45-60°C;
c) Rinsing;
d) Thermal disinfection;
e) Drying
**characterized in that**
the water is heated for step b) and step d) by recirculating it through the water heater (15) arranged outside and at a distance from the basin (3) and so not directly in contact with the basin (3), and the water heating during said ultrasonic cleansing step b) is mainly performed using the heating bands (18) and using water recirculation through the outside water heater (15) for the minimum necessary to mix the water and eliminate thermal stratification.

11. Method for ultrasonic cleansing and thermal disinfection in said ultrasonic thermal disinfector (1) according to claim 7, wherein the thermal disinfection step d) is performed keeping the water at a temperature of 80-93°C for at least 5 minutes of contact time.

12. Method for ultrasonic cleansing and thermal disinfection in the ultrasonic thermal disinfector (1) according to claims 7-9, wherein the drying step e) is performed by introducing air into the basin (3) through rotors or rotating nozzles (8).

## Patentansprüche

1. Wärmedesinfektor (1), der Folgendes umfasst:
- einen Deckel (2),
- ein Becken (3),
- mindestens einen Einlass für das Wasser (8, 9),
- einen Auslass (25) für das Wasser, der in die Kanalisation führt,
- einen Auslass (26) für das Wasser zur Rückführung, der dessen Wiedereinleiten in das Becken (3) gestattet,
- mindestens ein Element (24) zur Erzeugung von Ultraschall, eine Steuerelektronik,
**dadurch gekennzeichnet, dass** er Folgendes umfasst:
- ein außerhalb des Beckens (3) in einem Abstand zu diesem bzw. nicht in direktem Kontakt mit dem Becken (3) untergebrachtes Heizgerät (15)
- und mindestens ein an dem Becken (3) anliegendes Wärmeband (18).

2. Wärmedesinfektor nach Anspruch 1, bei dem das Wasser in das Becken (3) über einen oder zwei verschiedene Wege eintritt:
- das aus dem Netz kommende Wasser überquert eine Entkalkergruppe (21) und tritt über die Düse (9) in das Becken ein;
- das im Becken (3) enthaltene Wasser tritt aus dem Rückführungsausgang (26) aus, überquert das äußere Heizgerät (15) und tritt in das Becken über mindestens eine Spritzvorrichtung (8) ein; auf dieser genannten Strecke kann das Heizgerät (15) eingeschaltet sein und das Wasser erwärmen oder ausgeschaltet sein und es nicht erwärmen.

3. Wärmedesinfektor (1) nach Anspruch 1, bei dem mindestens einer (8) der Eingänge für das Wasser geeignet ist, eine mechanische Wirkung auf das Wasser auszuüben, wobei dieser Eingang (8) vorzugsweise die Form einer Drehdüse oder eines Rotors aufweist.

4. Wärmedesinfektor nach den Ansprüchen 1-3, der außerdem einen HEPA-Filter (27) zum Filtern der aus der Außenumgebung entnommenen Luft, eine Mündung (11) zum Einleiten der Luft in das Becken (3) und eine Mündung (28) zum Ableiten der Luft an die Außenumgebung umfasst.

5. Wärmedesinfektor nach dem vorangegangenen Anspruch, der Elemente zum Erwärmen der genannten von außen eingeleiteten Luft umfasst.

6. Wärmedesinfektor nach Anspruch 1, der außerdem ein Wärmetauschventil (29) umfasst, das es gestattet, die Luft zum Trocknen der Instrumente über die Drehdüsen oder die Rotoren (8) zu leiten.

7. Wärmedesinfektor nach einem oder mehreren der vorangegangenen Ansprüche, bei dem das genannte Becken (3) vorrangig in horizontaler Ebene verläuft.

8. Wärmedesinfektor nach dem vorangegangenen Anspruch, bei dem der genannte Ultraschallgenerator (24) vorzugsweise auf der Hauptverlaufsfläche des genannten Beckens (3) angeordnet ist, d. h., auf der unteren Fläche, die in der genannten horizontalen Ebene verläuft.

9. Wärmedesinfektor nach einem oder mehreren der vorangegangenen Ansprüche, der einen Behälter (2a) umfasst, in dem mindestens das genannte Becken (3) in höher gelegter Position untergebracht ist, und der außerdem Überflutungsschutzvorrichtungen (31) umfasst, die eine auf dem Boden des genannten Behälters (2a) angeordnete Auffangschale (32) umfassen, und einen an ein Wasserabsperrventil angeschlossenen Schwimmer (33), das geeignet ist, das Eintreten von Wasser in den Wärmedesinfektor (1) zu unterbinden, wenn der Schwimmer (33) aufgrund einer Überflutung nach oben steigt.

10. Methode für das Ultraschallwaschen und die Desinfektion mit Wärme der im Inneren des Beckens (3) des Wärmedesinfektors (1) enthaltenen Korbs (4) untergebrachten Instrumente, die die folgenden Schritte umfasst:
a) Vorspülen mit kaltem Wasser;
b) Waschen mit oder ohne Ultraschall der eingetauchten oder nicht eingetauchten Instrumente bei einer Temperatur von 45-60°C;
c) Spülen;
d) Wärmedesinfektion;
e) Trocknen
**dadurch gekennzeichnet, dass**
das Wasser für den Schritt b) und den Schritt d) erwärmt und über das Heizgerät (15) außerhalb des Beckens (3) und in einem Abstand zu diesem bzw. nicht in direktem Kontakt mit dem Becken (3) rückgeführt wird, und das Erwärmen des Wassers beim Schritt b) des Ultraschallwaschens der Instrumente vorwiegend unter Einsatz der Heizbänder (18) und Verwendung des Wasserrücklaufs über das externe Heizgerät (15) für das Mindestmaß erfolgt, das erforderlich ist, um das Wasser zu mischen und die Wärmeschichtbildung zu beseitigen.

11. Methode zum Ultraschallwaschen und Desinfizieren mit Wärme im Inneren des Ultraschall-Wärmedesinfektors (1) nach Anspruch 7, bei der der Schritt der Wärmedesinfektion d) ausgeführt wird, indem das Wasser für mindestens 5 Minuten Kontaktzeit bei einer Temperatur von 80-93°C gehalten wird.

12. Methode zum Ultraschallwaschen und die Desinfektion mit Wärme im Inneren des Ultraschall-Wärmedesinfektors (1) nach Anspruch 7-9, bei der der Schritt des Trocknens e) ausgeführt wird, indem die Luft über die Rotoren oder die Drehdüsen (8) in das Becken geleitet wird.

## Revendications

1. Désinfecteur thermique (1) comprenant :
- un couvercle (2),
- un bac (3),
- au moins une entrée pour l'eau (8, 9),
- une sortie (25) pour l'eau qui aboutit à l'égout,
- une sortie (26) d'eau pour la recirculation qui permet de la réintroduire dans le bac (3)
- au moins un moyen (24) pour la génération d'ultrasons, une électronique de contrôle,
**caractérisé en ce qu'**il comprend
- un réchauffeur (15) d'eau, installé à l'extérieur du bac (3) et éloigné de ce dernier, c'est-à-dire non en contact direct avec le bac 3,
- et au moins une bande (18) chauffante adhérant au bac (3).

2. Désinfecteur thermique selon la revendication 1, dans lequel l'eau entre dans le bac (3) en suivant un de deux chemins distincts :
- l'eau provenant du réseau passe à travers un groupe (21) décalcificateur et entre dans le bac par la buse (9) ;
- l'eau contenue dans le bac (3) sort par la sortie (26) de recirculation, passe à travers le réchauffeur (15) extérieur, entre dans le bac à travers au moins un gicleur (8) ; sur ledit chemin le réchauffeur (15) extérieur peut être allumé réchauffant l'eau ou éteint ne la réchauffant pas.

3. Désinfecteur thermique (1) selon la revendication 1, dans lequel au moins une (8) des entrées d'eau est apte à donner une action mécanique à l'eau, préférablement ladite entrée (8) a la forme d'une buse tournante ou d'une roue.

4. Désinfecteur thermique selon les revendications 1-3 comprenant en outre un filtre HEPA (27) pour filtrer l'air prélevé à l'extérieur, une bouche (11) pour l'introduction de l'air dans le bac (3) et une bouche (28) pour l'éjection de l'air à l'extérieur.

5. Désinfecteur thermique selon la revendication précédente comprenant des moyens de chauffage dudit air introduit de l'extérieur.

6. Désinfecteur thermique selon la revendication 1 comprenant en outre une soupape d'échange (29) permettant d'acheminer l'air pour le séchage des instruments à travers les buses rotatives ou les roues (8).

7. Désinfecteur thermique selon l'une ou plusieurs des revendications précédentes, dans lequel ladite cuve (3) s'étend principalement sur le plan horizontal.

8. Désinfecteur thermique selon la revendication précédente, dans lequel ledit générateur d'ultrasons (24) est placé, de préférence, au niveau de la surface de plus grande extension dudit bac (3), c'est-à-dire au niveau de la surface inférieure qui s'étend sur ledit plan horizontal.

9. Désinfecteur thermique, selon une ou plusieurs des revendications précédentes, comprenant un boîtier (2a) dans lequel est logé au moins ledit bac (3) en position relevée, comprenant en outre des moyens anti-inondation (31), comprenant un plateau de retenue (32) situé sur le fond dudit boîtier (2a) et comprenant un flotteur (33) branché sur une soupape d'arrêt de l'eau, apte à empêcher l'entrée d'eau dans le désinfecteur thermique (1) quand le flotteur (33) monte à cause d'une inondation.

10. Procédé pour le lavage à ultrasons et la désinfection par la chaleur des instruments placés dans le panier (4) contenu à l'intérieur du bac (3) du désinfecteur thermique (1), comprenant les phases suivantes :
a) prélavage à l'eau froide ;
b) lavage avec ou sans ultrasons des instruments immergés ou non immergés à une température de 45-60 °C ;
c) rinçage ;
d) désinfection thermique ;
e) séchage
**caractérisé en ce que**
l'eau est chauffée pour la phase b) et la phase d) en la faisant recirculer à travers le réchauffeur (15) à l'extérieur du bac (3) et éloigné de ce dernier, c'est-à-dire non en contact direct avec le bac (3), et le chauffage de l'eau dans la phase b) de lavage à ultrasons des instruments est effectuée en utilisant principalement les bandes (18) chauffantes et en utilisant la recirculation de l'eau à travers le réchauffeur (15) extérieur pour le minimum nécessaire à mélanger l'eau et à éliminer la stratification thermique.

11. Procédé de lavage à ultrasons et désinfection par la chaleur effectué à l'intérieur du désinfecteur thermique (1) ultrasonore selon la revendication 7, dans lequel la phase de désinfection thermique d) est effectuée en maintenant l'eau à une température de 80-93 °C pour au moins 5 minutes de durée de contact.

12. Procédé de lavage à ultrasons et désinfection par la chaleur effectué à l'intérieur du désinfecteur thermique (1) ultrasonore selon les revendications 7-9, dans lequel la phase de séchage e) est effectuée en introduisant l'air dans le bac (3) à travers les roues ou les buses rotatives (8).
